(19) Europäisches Patentamt
European Patent Office
Office européen des brevets

(11) Veröffentlichungsnummer: 0 222 254 A2

(12) **EUROPÄISCHE PATENTANMELDUNG**

(21) Anmeldenummer: 86114990.4

(22) Anmeldetag: 28.10.86

(51) Int. Cl.4: C07D 401/04 , C07D 413/04 , A01N 43/56 , A01N 43/74 , A01N 43/50 , //C07D213/80

(30) Priorität: 09.11.85 DE 3539809
11.07.86 DE 3623302

(43) Veröffentlichungstag der Anmeldung: 20.05.87 Patentblatt 87/21

(84) Benannte Vertragsstaaten: AT BE CH DE FR GB IT LI NL

(71) Anmelder: BAYER AG
Konzernverwaltung RP Patentabteilung
D-5090 Leverkusen 1 Bayerwerk(DE)

(72) Erfinder: Thomas, Rudolf, Dr.
Dellbusch 269
D-5600 Wuppertal 2(DE)
Erfinder: Babczinski, Peter, Dr.
In der Lohrenbeck 11
D-5600 Wuppertal 1(DE)
Erfinder: Santel, Hans-Joachim, Dr.
Grünstrasse 9a
D-5090 Leverkusen 1(DE)
Erfinder: Eue, Ludwig, Dr.
Paul-Klee-Strasse 36
D-5090 Leverkusen 1(DE)
Erfinder: Schmidt, Robert R., Dr.
Im Waldwinkel 110
D-5060 Bergisch Gladbach 2(DE)

(54) **Nicotinsäurederivate.**

(57) Die Erfindung betrifft neue Nicotinsäurederivate der Formel (I),

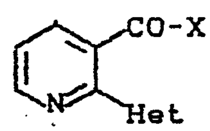

(I)

in welcher
X für Hydroxy, Alkoxy oder den Rest -OM steht, wobei
M für ein Alkali-oder Erdalkaliionenäquivalent oder ein gegebenenfalls substituiertes Ammoniumion steht,
und
Het für jeweils substituiertes Isoxazolyl, Pyrimidinyl oder Pyrazolyl steht,
mehrere Verfahren zu ihrer Herstellung und ihre Verwendung als Herbizide sowie neue Zwischenprodukte.

EP 0 222 254 A2

Xerox Copy Centre

## Nicotinsäurederivate

Die Erfindung betrifft neue Nicotinsäurederivate, mehrere Verfahren zu ihrer Herstellung und ihre Verwendung als Herbizide.

Es ist bereits bekannt, daß bestimmte herterosubstituierte Benzoesäurederivate pflanzenwachstumsregulierende Eigenschaften besitzen und daß bestimmte Imidazolin-nicotinsäure-Derivate herbizide Eigenschaften besitzen (vgl. DE-OS 26 00 655 und EP-A-41 623). Ihre Wirkung ist jedoch nicht immer in allen Anwendungsbereichen völlig zufriedenstellend.

Es wurden neue Nicotinsäurederivate der allgemeinen Formel (I)

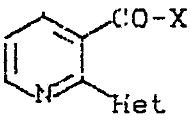

(I)

in welcher

X für Hydroxy, Alkoxy oder den Rest -OM steht, wobei

M für ein Alkali-oder Erdalkaliionenäquivalent oder ein gegebenenfalls substituiertes Ammoniumion steht, und

Het für jeweils substituiertes Isoxazolyl, Pyrimidinyl oder Pyrazolyl steht, wobei jeweils als Substituenten Alkyl, Alkenyl, Alkoxy, Alkylthio, Alkylamino, Hydroxy, Mercapto, Amino, gegebenenfalls durch Halogen oder Alkoxy substituiertes Alkylcarbonyl, Cycloalkyl sowie jeweils gegebenenfalls substituiertes Phenyl oder Naphthyl in Frage kommen, weiterhin Pyrazolyl einen ankondensierten gegebenenfalls substituierten Cycloalkylrest enthalten kann,

gefunden.

Weiterhin wurde gefunden, daß man die neuen Nicotinsäurederivate der Formel (I),

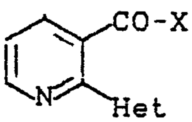

(I)

in welcher

X für Hydroxy, Alkoxy oder den Rest -OM steht, wobei

M für ein Alkali-oder Erdalkaliionenäquivalent oder ein gegebenenfalls substituiertes Ammoniumion steht, und

Het für jeweils substituiertes Isoxazolyl, Pyrimidinyl oder Pyrazolyl steht, wobei jeweils als Substituenten Alkyl, Alkenyl, Alkoxy, Alkylthio, Alkyl amino, Hydroxy, Mercapto, Amino, gegebenenfalls durch Halogen oder Alkoxy substituiertes Alkylcarbonyl, Cycloalkyl sowie jeweils gegebenenfalls substituiertes Phenyl oder Naphthyl in Frage kommen, weiterhin Pyrazolyl einen ankondensierten gegebenenfalls substituierten Cycloalkylrest enthalten kann,

erhält, wenn man

A) Diketone der Formel (IIa),

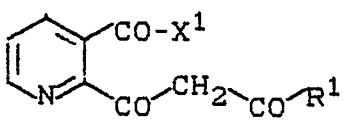

(IIa)

in welcher

$X^1$ für Alkoxy steht und

$R^1$ für Alkyl, Alkenyl, Cycloalkyl, Acetyl sowie für gegebenenfalls substituiertes Phenyl oder Naphthyl steht,

a) mit Hydroxylamin bzw. dessen Salze zu den Nicotinsäurederivaten der Formel (Ia)

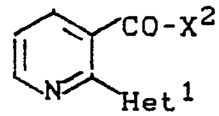

(I a)

in welcher

$X^2$ für Hydroxy oder Alkoxy steht, und

$Het^1$ für substituiertes Isoxazolyl steht, wobei als Substituenten Alkyl, Alkenyl, Cycloalkyl, Acetyl sowie jeweils gegebenenfalls substuiertes Phenyl oder Naphthyl in Frage kommen,

oder

b) mit Amidinen der allgemeinen Formel (III), bzw. deren Salze

$R^2-C(=NH)-NH_2$ (III)

in welcher

$R^2$ für Alkyl, Alkoxy, Alkylthio, Alkylamino oder gegebenenfalls substituiertes Phenyl steht,

zu den Nicotinsäurederivaten der Formel (Ib),

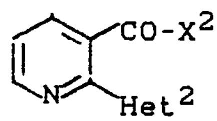

(I b)

in welcher

$X^2$ die oben angegebene Bedeutung hat und

$Het^2$ für substituiertes Pyrimidinyl steht, wobei als Substituenten Alkyl, Alkenyl, Cycloalkyl, Alkoxy, Alkylthio, Alkylamino, Acetyl sowie jeweils gegebenenfalls substituiertes Phenyl oder Naphthyl in Frage kommen,

oder

c) mit Harnstoffderivaten der allgemeinen Formel (IV), bzw. deren Salze

$NH_2-C(=Y)-NH_2$ (IV)

in welcher

Y für Sauerstoff, Schwefel oder den Rest NH steht

zu den Nicotinsäurederivaten der Formel (Ic)

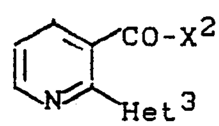

(I c)

in welcher

$X^2$ die oben angegebene Bedeutung hat, und

$Het^3$ für substituiertes Pyrimidinyl steht, wobei als Substituenten Alkyl, Alkenyl, Cycloalkyl, Hydroxy, Mercapto, Amino, Acetyl sowie jeweils gegebenenfalls substituiertes Phenyl oder Naphthyl in Frage kommen, in Gegenwart eines Verdünnungsmittels umsetzt oder

B) gegebenenfalls die nach Verfahren A) erhaltenen Nicotinsäurederivate der Formel (Ie)

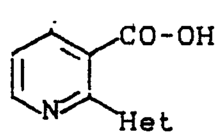

(Ie)

in welcher
Het die oben angegebene Bedeutung hat,
in üblicher Weise mit einer Base in Gegenwart eines Lösungsmittels zu den Nicotinsäurederivaten der Formel (If)

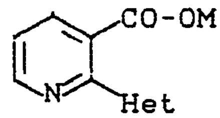

(If)

in welcher
M und Het die oben angegebenen Bedeutungen haben, umsetzt und

C) 1,3-Diketone der Formel (II)

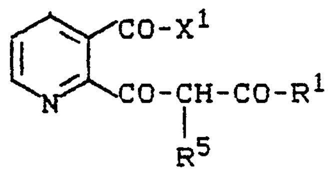

(II)

in welcher
$X^1$ für Alkoxy steht,
$R^1$ für Alkyl, Alkenyl, Cycloalkyl, Acetyl sowie für jeweils gegebenenfalls substituiertes Phenyl oder Naphthyl steht und
$R^5$ für Wasserstoff steht oder
$R^1$ und $R^5$ gemeinsam für eine gegebenenfalls substituierte Alkylenkette stehen,
mit Hydrazinen der allgemeinen Formel (V)
$R^4-NH-NH_2$ (V)
in welcher
$R^4$ für Wasserstoff, Alkyl, gegebenenfalls durch Halogen oder Alkoxy substituiertes Alkylcarbonyl oder gegebenenfalls subsituiertes Phenyl steht,
zu den Nicotinsäurederivaten der Formel (Id)

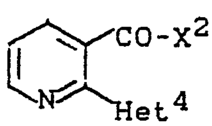

(I d)

in welcher
$X^2$ die oben angegebene Bedeutung hat, und
$Het^4$ für substituiertes Pyrazolyl steht, wobei als Substituenten Alkyl, Alkenyl, Cycloalkyl, gegebenenfalls durch Halogen oder Alkoxy substituiertes Alkylcarbonyl sowie jeweils gegebenenfalls substituiertes Phenyl oder Naphthyl in Frage kommen, weiterhin Pyrazolyl einen ankondensierten gegebenenfalls substituierten Cycloalkylrest enthalten kann,
in Gegenwart eines Verdünnungsmittels umsetzt.

Schließlich wurde gefunden, daß die neuen Nicotinsäurederivate der allgemeinen Formel (I) herbizide Eigenschaften besitzen.

Die erfindungsgemäßen Nicotinsäurederivate sind durch die Formel (I) allgemein definiert. Bevorzugt sind Verbindungen der Formel (I),
in welcher
X für Hydroxy, für Alkoxy mit 1 bis 6 Kohlenstoffatomen, oder für den Rest -OM steht, wobei

M für ein Natrium-oder Kaliumion, für ein Magnesium-oder Calciumionenäquivalent oder für ein Ammonium-, Mono-, Di-, Tri-oder Tetraalkylammoniumion mit jeweils 1 bis 4 Kohlenstoffatomen in den einzelnen Alkylresten steht, und

Het für

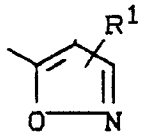 , 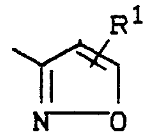 , 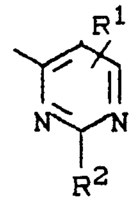 , 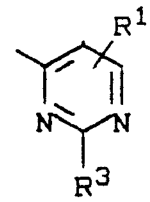 ,

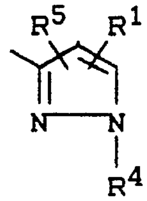 oder 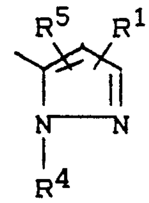

steht, wobei

$R^1$ für geradkettiges oder verzweigtes Alkyl mit 1 bis 6 Kohlenstoffatomen, für Alkenyl mit 2 bis 6 Kohlenstoffatomen, für Cycloalkyl mit 3 bis 6 Kohlenstoffatomen, für Acetyl sowie für jeweils gegebenenfalls einfach bis dreifach, gleich oder verschieden substituiertes Phenyl oder Naphthyl steht, wobei als Substituenten in Frage kommen:

Halogen, jeweils geradkettiges oder verzweigtes Alkyl, Alkoxy, Alkylthio und Dialkylamino mit 1 bis 4 Kohlenstoff atomen in den einzelnen Alkylteilen, Halogenalkyl mit 1 oder 2 Kohlenstoffatomen und 1 bis 5 gleichen oder verschiedenen Halogenatomen wie Fluor-und Chloratomen, Nitro und Phenyl,

$R^2$ für jeweils geradkettiges oder verzweigtes Alkyl, Alkoxy, Alkylthio oder Alkylamino mit 1 bis 6 Kohlenstoffatomen oder für gegebenenfalls einfach bis dreifach, gleich oder verschieden substituiertes Phenyl steht, wobei als Substituenten Halogen, geradkettiges oder verzweigtes Alkyl und Alkoxy mit jeweils 1 bis 4 Kohlenstoffatomen und Halogenalkyl mit 1 oder 2 Kohlenstoffatomen und 1 bis 5 gleichen oder verschiedenen Halogenatomen wie Fluor-und Chloratomen in Frage kommen,

$R^3$ für Hydroxy, Mercapto oder für Amino steht,

$R^4$ für Wasserstoff, geradkettiges oder verzweigtes Alkyl mit 1 bis 6 Kohlenstoffatomen, gegebenenfalls durch Fluor, Chlor und/oder Brom oder $C_1$-$C_4$-Alkoxy substituiertes $C_1$-$C_4$-Alkylcarbonyl oder für gegebenenfalls einfach bis dreifach, gleich oder verschieden substituiertes Phenyl steht, wobei als Substituenten Halogen, geradkettiges oder verzweigtes Alkyl und Alkoxy mit jeweils 1 bis 4 Koh lenstoffatomen und Halogenalkyl mit 1 oder 2 Kohlenstoffatomen und 1 bis 5 gleichen oder verschiedenen Halogenatomen wie Fluor-und Chloratomen in Frage kommen,

$R^5$ für Wasserstoff steht oder

$R^1$ und $R^5$ gemeinsam für eine gegebenenfalls benzannellierte Alkylenkette mit 3 bis 5 Kohlenstoffatomen stehen.

Besonders bevorzugt sind Verbindungen der Formel (I), in denen

X für Hydroxy, Methoxy, Ethoxy, n-oder i-Propoxy oder für den Rest -OM steht, wobei

M für Natrium-, Kalium-, Ammonium-oder Tetraalkylammoniumion mit 1 bis 4 Kohlenstoffatomen in den einzelnen Alkylresten steht, und

Het für

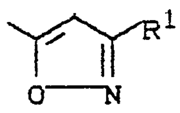 , 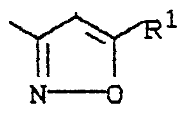 , 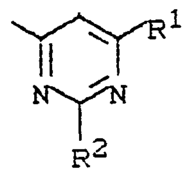 , 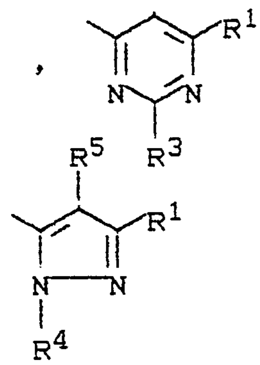 ,

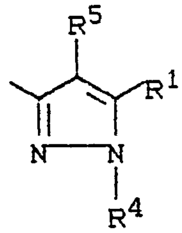 oder

steht, wobei

$R^1$ für Methyl, Ethyl, n-oder i-Propyl, n-, i-, s-oder t-Butyl, für Vinyl oder Isopropyliden, für Cyclopropyl, Cylcopentyl, Cyclohexyl, Acetyl sowie für jeweils gegebenenfalls einfach oder zweifach, gleich oder verschieden substituiertes Phenyl oder Naphthyl steht, wobei als Substituenten in Frage kommen:

Fluor, Chlor, Brom, Iod, Methyl, Ethyl, n-und i-Propyl, Methoxy, Ethoxy, n-und i-Propoxy, Methylthio, Ethylthio, n-und i-Propylthio, Dimethylamino, Diethylamino,Dipropylamino, Trifluormethyl, Nitro und Phenyl,

$R^2$ für Methyl, Ethyl, n-oder i-Propyl, n-, i-, s-oder t-Butyl, Methoxy, Ethoxy, n-oder i-Propoxy, n-, i-, s-oder t-Butoxy, Methylthio, Ethylthio, n-oder i-Propylthio, n-, i-, s-oder t-Butylthio, Methylamino, Ethylamino, n-und i-Propylamino sowie für gegebenenfalls einfach oder zweifach, gleich oder verschieden substituiertes Phenyl steht, wobei als Substituenten in Frage kommen:

Fluor, Chlor, Brom, Methyl, Ethyl n-und i-Propyl, Methoxy, Ethoxy, n-und i-Propoxy und Trifluormethyl,

$R^3$ für Hydroxy, Mercapto oder Amino steht, und

$R^4$ für Wasserstoff, Methyl, Ethyl, n-oder i-Propyl, n-, i-, s-oder t-Butyl, für Acetyl, Chlormethylcarbonyl, Chlorethylcarbonyl, Methoxymethylcarbonyl, Ethoxymethylcarbonyl, Methoxyethylcarbonyl, Ethoxyethylcarbonyl, sowie für gegebenenfalls einfach oder zweifach, gleich oder verschieden substituiertes Phenyl steht, wobei als Substituenten in Frage kommen:

Fluor, Chlor, Brom, Methyl, Ethyl, n-und i-Propyl, Methoxy, Ethoxy, n-und i-Propoxy und Trifluormethyl steht,

$R^5$ für Wasserstoff steht oder

$R^1$ und $R^5$ gemeinsam für eine gegebenenfalls benzannellierte Alkylenkette mit 3 oder 4 Kohlenstoffatomen stehen.

Ganz besonders bevorzugt ist dabei die Gruppe von Verbindungen der Formel (I), in welcher

X für Hydroxy, Methoxy, Ethoxy, n-oder i-Propoxy oder für den Rest -OM steht, wobei

M für Natrium-, Kalium-, Ammonium-oder Tetraalkylammoniumion mit 1 bis 4 Kohlenstoffatomen in den einzelnen Alkylresten steht, und

Het für

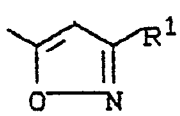 oder 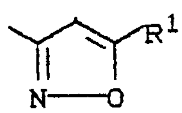

steht, wobei

$R^1$ für Methyl, Ethyl, n-oder i-Propyl, n-, i-, s-oder t-Butyl, für Vinyl oder Isopropyliden, für Cyclopropyl, Cylcopentyl, Cyclohexyl, Acetyl sowie für jeweils gegebenenfalls einfach oder zweifach, gleich oder verschieden substituiertes Phenyl oder Naphthyl steht, wobei als Substituenten in Frage kommen:

Fluor, Chlor, Brom, Iod, Methyl, Ethyl, n-und i-Propyl, Methoxy, Ethoxy, n-und i-Propoxy, Methylthio, Ethylthio, n-und i-Propylthio, Dimethylamino, Diethylamino,Dipropylamino, Trifluormethyl, Nitro und Phenyl.

Eine weitere ganz besonders bevorzugte Gruppe sind Verbindungen der Formel (I), in denen

X für Hydroxy, Methoxy, Ethoxy, n-oder i-Propoxy oder für den Rest -OM steht, wobei

M für Natrium-, Kalium-, Ammonium-oder Tetraalkylammoniumion mit 1 bis 4 Kohlenstoffatomen in den einzelnen Alkylresten steht, und

Het für

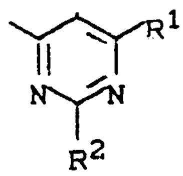 oder 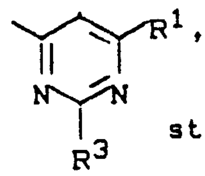 steht,

wobei

$R^1$ für Methyl, Ethyl, n-oder i-Propyl, n-, i-, s-oder t-Butyl, für Vinyl oder Isopropyliden, für Cyclopropyl, Cyclopentyl, Cyclohexyl, Acetyl sowie für jeweils gegebenenfalls einfach oder zweifach, gleich oder verschieden substituiertes Phenyl oder Naphthyl steht, wobei als Substituenten in Frage kommen:

Fluor, Chlor, Brom, Iod, Methyl, Ethyl, n-und i-Propyl, Methoxy, Ethoxy, n-und i-Propoxy, Methylthio, Ethylthio, n-und i-Propylthio, Dimethylamino, Diethylamino, Dipropylamino, Trifluormethyl, Nitro und Phenyl,

$R^2$ für Methyl, Ethyl, n-oder i-Propyl, n-, i-, s-oder t-Butyl, Methoxy, Ethoxy, n-oder i-Propoxy, n-, i-, s-oder t-Butoxy, Methylthio, Ethylthio, n-oder i-Propylthio, n-, i-, s-oder t-Butylthio, Methylamino, Ethylamino, n-und i-Propylamino sowie für gegebenenfalls einfach oder zweifach, gleich oder verschieden substituiertes Phenyl steht, wobei als Substituenten in Frage kommen:

Fluor, Chlor, Brom, Methyl, Ethyl n-und i-Propyl, Methoxy, Ethoxy, n-und i-Propoxy und Trifluormethyl, und

$R^3$ für Hydroxy, Mercapto oder Amino steht.

Eine andere ganz besonders bevorzugte Gruppe sind Verbindungen der Formel (I), in denen

X für Hydroxy, Methoxy, Ethoxy, n-oder i-Propoxy oder für den Rest -OM steht, wobei

M für Natrium-, Kalium-, Ammonium-oder Tetraalkylammoniumion mit 1 bis 4 Kohlenstoffatomen in den einzelnen Alkylresten steht, und

Het für

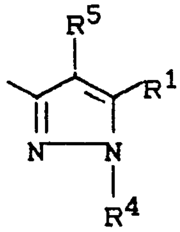 oder 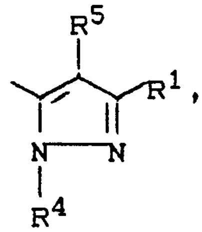

steht, wobei

$R^1$ für Methyl, Ethyl, n-oder i-Propyl, n-, i-, s-oder t-Butyl, für Vinyl oder Isopropyliden, für Cyclopropyl, Cylcopentyl, Cyclohexyl, Acetyl sowie für jeweils gegebenenfalls einfach oder zweifach, gleich oder verschieden substituiertes Phenyl oder Naphthyl steht, wobei als Substituenten in Frage kommen:

Fluor, Chlor, Brom, Iod, Methyl, Ethyl, n-und i-Propyl, Methoxy, Ethoxy, n-und i-Propoxy, Methylthio, Ethylthio, n-und i-Propylthio, Dimethylamino, Diethylamino,Dipropylamino, Trifluormethyl, Nitro und Phenyl und

$R^4$ für Wasserstoff, Methyl, Ethyl, n-oder i-Propyl, n-, i-, s-oder t-Butyl, für Acetyl, Chlormethylcarbonyl, Methoxymethylcarbonyl sowie für gegebenenfalls einfach oder zweifach, gleich oder verschieden substituiertes Phenyl steht, wobei als Substituenten in Frage kommen:

Fluor, Chlor, Brom, Methyl, Ethyl, n-und i-Propyl, Methoxy, Ethoxy, n-und i-Propoxy und Trifluormethyl steht,

$R^5$ für Wasserstoff steht oder

$R^1$ und $R^5$ gemeinsam für eine gegebenenfalls benzannellierte Alkylenkette mit 3 oder 4 Kohlenstoffatomen stehen.

Verwendet man beispielsweise 2-(1,3-Dioxo-3-(t-butyl)-prop-1-yl)-3-methoxycarbonyl-pyridin und Hydroxylaminhydrochlorid als Ausgangsstoffe, so läßt sich der Reaktionsablauf des erfindungsgemäßen Verfahrens (A-a) durch das folgende Formelschema darstellen:

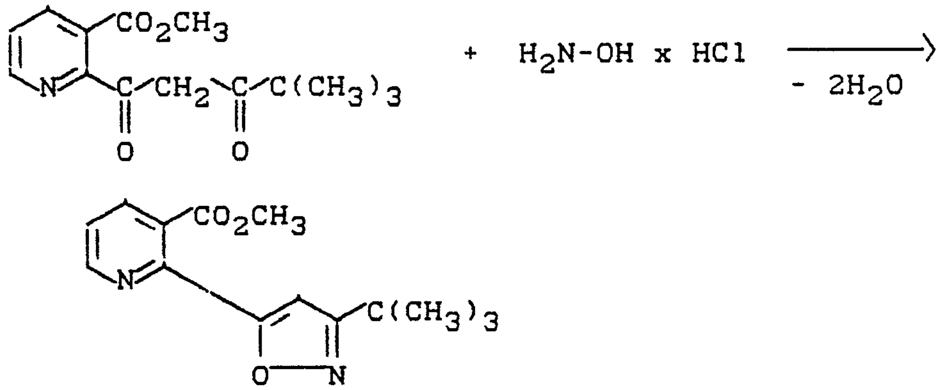


Verwendet man beispielsweise 2-(1,3-Dioxo-3-phenyl-prop-1-yl)-3-methoxycarbonyl-pyridin und Isobutyrylamidinhydrochlorid als Ausgangsstoffe, so läßt sich der Reaktionsablauf des erfindungsgemäßen Verfahrens (A-b) durch das folgende Formelschema darstellen:

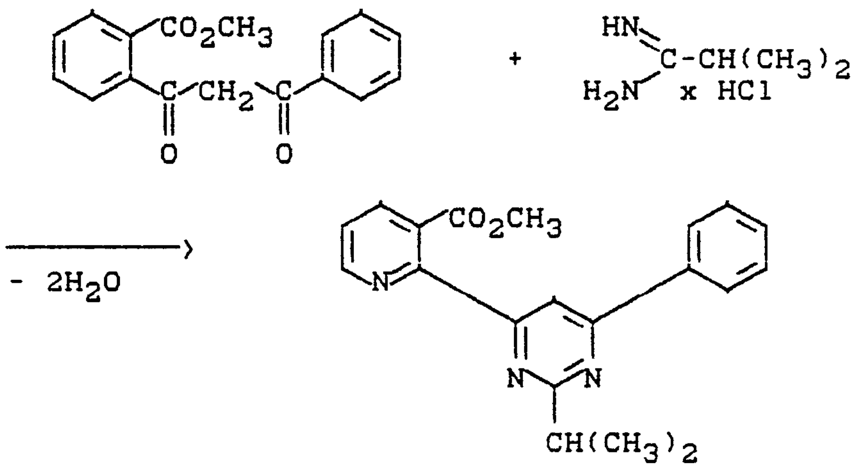


Verwendet man beispielsweise 2-(1,3-Dioxo-3-phenyl-prop-1-yl)-3-methoxycarbonylpyridin und Harnstoff als Ausgangsstoffe, so läßt sich der Reaktionsablauf des erfindungsgemäßen Verfahrens (A-c) durch das folgende Formelschema darstellen:

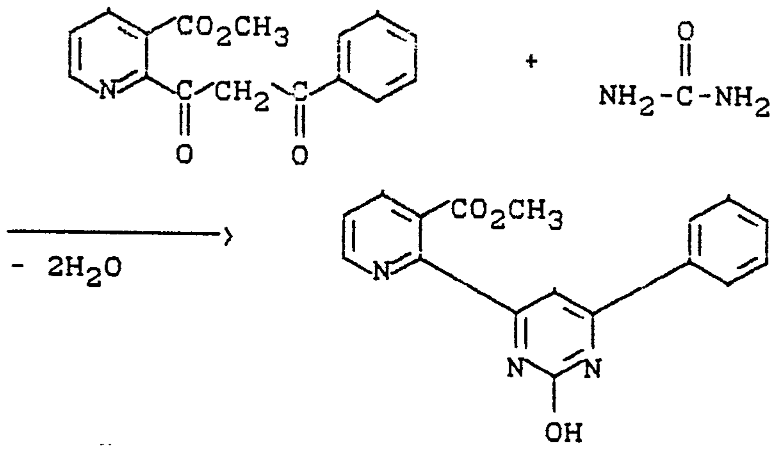

Verwendet man beispielsweise 2-(5-Isopropyl-pyrazol-3-yl)-3-carbonsäure-pyridin und Calciumcarbonat als Ausgangsstoffe, so läßt sich der Reaktionsablauf des erfindungsgemäßen Verfahrens (B) durch das folgende Formelschema darstellen:

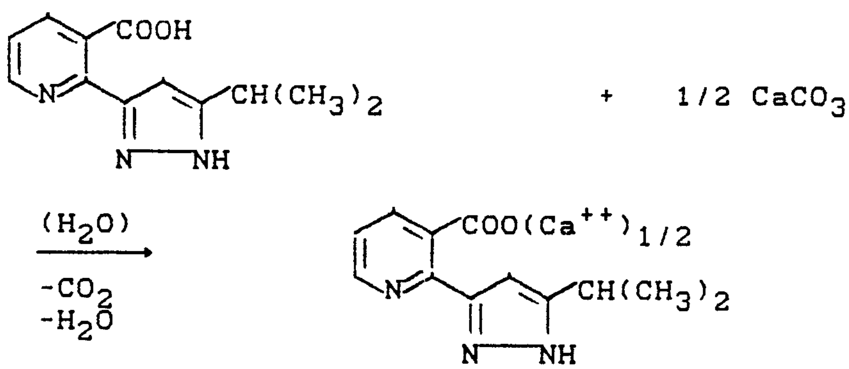


Verwendet man beispielsweise 2-(1,3-Dioxo-3-(4-methoxyphenyl)-prop-1-yl)-3-methoxycarbonyl-pyridin und Methylhydrazin als Ausgangsstoffe, so läßt sich der Reaktionsablauf des erfindungsgemäßen Verfahrens (C) durch das folgende Formelschema darstellen:

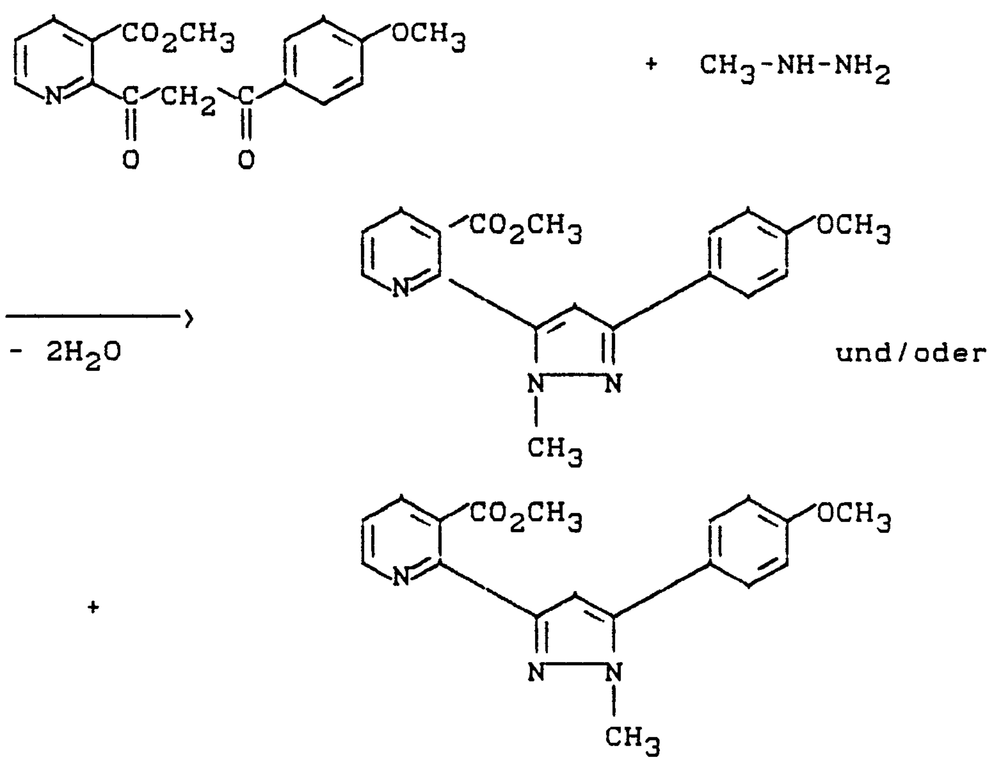


Die zur Durchführung der erfindungsgemäßen Verfahren A) und C) als Ausgangsstoffe benötigten Diketone sind durch die Formeln (II) und (IIa) allgemein definiert. In den Formeln (II) und (IIa) stehen $R^1$ und $R^5$ vorzugsweise für diejenigen Reste, die bereits im Zusammenhang mit der Beschreibung der erfindungsgemäßen Stoffe der Formel (I) als bevorzugt für diese Substituenten genannt wurden und $X^1$ steht vorzugsweise für Alkoxy mit 1 bis 6 Kohlenstoffatomen und besonders bevorzugt für Methoxy, Ethoxy sowie n-oder i-Propoxy.

Die Diketone der Formeln (II) und (IIa) und ihre entsprechenden Enolformen sind noch nich bekannt; sie können jedoch in allgemein bekannter Art und Weise erhalten werden, indem man z. B. Ketone der Formel (VI)

$R^5-CH_2-\overset{O}{\overset{\|}{C}}-R^1$ (VI)

in welcher

$R^1$ und $R^5$ die oben angegebene Bedeutung haben,

mit 2,3-Dialkoxycarbonyl-pyridinen der Formel (VII)

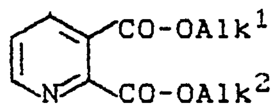

(VII)

in welcher

$Alk^1$ und $Alk^2$ gleich oder verschieden sein können und für Alkyl, bevorzugt für Alkyl mit 1 bis 6 Kohlenstoffatomen und besonders bevorzugt für Methyl, Ethyl sowie n-oder i-Propyl stehen,

in Gegenwart einer starken Base wie z. B. Natriumhydrid, Natriumamid oder Natriumalkoholaten wie Natriummethylat oder Natriumethylat und in Gegenwart eines inerten organischen Lösungsmittels wie z. B. Toluol, Xylol, Dichlorbenzol oder Cyclohexan bei Temperaturen zwischen 80 °C und 150 °C umsetzt.

Die Ketone der Formel (VI) und die 2,3-Dialkoxycarbonyl-pyridine der Formel (VII) sind allgemein bekannte Verbindungen der organischen Chemie.

Die zur Durchführung der erfindungsgemäßen Verfahren A) und C) weiterhin als Ausgangsstoffe benötigten Amidine, Harnstoffderivate bzw. Hydrazine sind durch die Formel (III), (IV) bzw. (V) allgemein definiert. In der Formel (III) bzw. (V) steht $R^2$ bzw. $R^4$ vorzugsweise für den Rest, der bereits im Zusammenhang mit der Beschreibung der erfindungsgemäßen Stoffe der Formel (I) als bevorzugt für diesen Substituenten genannt wurde. In der Formel (IV) steht Y vorzugsweise für Sauerstoff, Schwefel oder den Rest NH.

Das Hydroxylamin sowie die Amidine der Formel (III) und die Harnstoffderivate der Formel (IV) werden bevorzugt in Form ihrer Salze eingesetzt, wie beispielsweise als Hydrohalogenide oder Hydrosulfate.

Hydroxylamin bzw. dessen Salze, Amidine der Formel (III) bzw. deren Salze, Harnstoffderivate der Formel (IV) bzw. deren Salze, sowie Hydrazine der Formel (V) sind allgemein bekannte Verbindungen der organischen Chemie.

Zur Durchführung der erfindungsgemäßen Verfahren A) und C) kommen als Verdünnungsmittel inerte, nicht mit Wasser mischbare Lösungsmittel, wie z.B. aromatische Kohlenwasserstoffe, wie insbesondere Toluol oder Benzol, Chlorkohlenwasserstoffe, wie insbesondere Tetrachlormethan, oder wasserbindende Solventien in Frage, wie insbesondere ein Gemisch aus Eisessig und Natriumacetat.

Bei der Verwendung des Gemisches aus Eisessig und Natriumacetat bleibt bei kurzzeitigem Erhitzen der Ester-Rest in der 3-Position erhalten, während bei längerem Erhitzen bevorzugt die freie Säure gebildet wird.

Die Reaktionstemperaturen können bei der Durchführung der erfindungsgemäßen Verfahren A) und C) in einem größeren Bereich variiert werden. Im allgemeinen arbeitet man bei Temperaturen zwischen 0 °C und 140 °C, vorzugsweise bei Temperaturen zwischen 20 °C und 120 °C.

Zur Durchführung der erfindungsgemäßen Verfahren A) und C) werden die benötigten Ausgangsstoffe im allgemeinen in angenähert äquimolaren Mengenverhältnissen eingesetzt. Es ist jedoch auch möglich, eine der beiden jeweils eingesetzten Komponenten in einem Überschuß zu verwenden.

Die Aufarbeitung erfolgt bei den erfindungsgemäßen Verfahren A) und C) nach üblichen Methoden.

Die für die Durchführung des erfindungsgemäßen Verfahrens B) als Ausgangsstoffe zu verwendenden Nicotinsäurederivate der Formel (I e) sind erfindungsgemäße Verbindungen und können gemäß Verfahren A) hergestellt werden.

Als Basen kommen für das erfindungsgemäße Verfahren B) alle üblicherweise verwendbaren Alkali-, Erdalkali-und Ammoniumsalze sowie Mono-, Di-und Trialkylamine in Frage. Hierzu gehören vorzugsweise Kaliumcarbonat, Natriumcarbonat, Calciumcarbonat, Magnesiumcarbonat, Ammoniumcarbonat, Diethylamin und Triethylamin.

Als Lösungsmittel kommen für die erfindungsgemäße Umsetzung B) vorzugsweise mit Wasser mischbare organische Lösungsmittel in Frage. Hierzu gehören vorzugsweise Alkohole wie Methanol, Ethanol, Propanol und Ether wie Tetrahydrofuran.

Die Reaktionstemperaturen können bei der Durchführung des erfindungsgemäßen Verfahrens B) in einem größeren Bereich variiert werden. Im allgemeinen arbeitet man bei Temperaturen zwischen -30 °C und 100 °C, vorzugsweise zwischen 0 °C und 60 °C.

Die Umsetzung erfolgt bei dem erfindungsgemäßen Verfahren B) in üblicher Art und Weise, indem man Lösungen der Reaktionspartner in äquimolaren Mengen mischt und die dabei ausfallenden Salze durch Abfiltrieren oder, falls keine Fällung eintritt, durch Eindampfen der Lösung isoliert und sie gegebenenfalls durch Umkristallisieren reinigt.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea,Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie-und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht-und Hopfenanlagen und zur selektiven Unkrautbekämpfung in einjährigen Kulturen eingesetzt werden.

Darüberhinaus zeigen die erfindungsgemäßen Wirkstoffe in entsprechenden Aufwandmengen bzw. Konzentrationen auch fungizide Wirksamkeit, wie insbesondere gegen Oomyceten und Pyricularia oryzae am Reis.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoffimprägnierte Natur-und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder - schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphtaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Ge steine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln: als Emulgier-und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate: als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo-und Metallphthalocyaninfarbstoffe und Spurennährstoffe wie Salze von Eisen, Mangan, Bor, Kupfer, Kobald, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Auch Mischungen mit anderen bekannten Wirkstoffen, wie Fungiziden, Insektiziden, Akariziden, Nematiziden, Schutzstoffe gegen Vogelfraß, Pflanzennährstoffe und Bodenstrukturverbesserungsmittel sind mglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

Herstellungsbeispeile:

Beispiel 1:

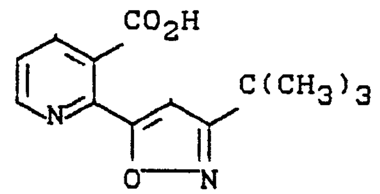

(I-1)

(Verfahren A-a)

7,9 g (0,06 Mol) 2-(1,3-Dioxo-3-t-butyl-prop-1-yl)-3-methoxycarbonylpyridin, 4,1 g (0,06 Mol) Hydroxylaminhydrochlorid und 6 g Natriumacetat werden in 120 ml Eisessig 3 Stunden unter Rückfluß erhitzt. Anschließend wird die Reaktionsmischung in 1 l Wasser gegossen und mehrfach mit insgesamt 300 ml Essigsäureethylester extrahiert. Nach dem Trocknen der organischen Phase über Natriumsulfat wird diese im Vakuum eingedampft. Der Rückstand wird in Portionen von insgesamt 100 ml Wasser ausgekocht, die wäßrigen Extrakte auf 10 °C abgekühlt, die ausfallenden farblosen Kristalle abgesaugt und im Vakuum getrocknet.

Man erhält 4,2 g (57 % der Theorie) 2-(3-t-Butyl-isoxazol-5-yl)-3-carbonsäure-pyridin als farblose Kristalle vom Schmelzpunkt 113 °C.

Beispiel 2:

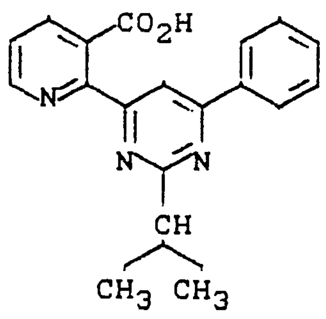

(I - 2)

(Verfahren A-b)

14,2 g (0,05 Mol) 2-(1,3-Dioxo-3-phenyl-prop-1-yl)-3-methoxycarbonyl-pyridin, 12,3 g (0,1 Mol) Isobutyrylamidinhydrochlorid und 10 g Natriumacetat werden in 200 ml Eisessig 3 Stunden unter Rückfluß erhitzt. Anschließend wird das Reaktionsgemisch in 1,5 l Wasser eingerührt, mehrfach mit insgesamt 2 l Essigsäureethylester extrahiert, die Extrakte über Natriumsulfat getrocknet und im Vakuum eingedampft. Der Rückstand wird über Kieselgel mit Chloroform/Essigsäureethylester (V/V = 1:1) chromatographiert.

Man erhält 0,9 g (6 % der Theorie) 2-(2-Isopropyl-6-phenyl-pyrimidin-4-yl)-3-carbonsäure-pyridin als farblose Kristalle vom Schmelzpunkt 176 °C (Zersetzung).

Herstellung des Ausgangsproduktes:

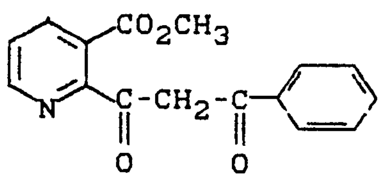

(II-1)

Eine Lösung von 19,5 g (0,1 Mol) 2,3-Dimethoxycarbonyl-pyridin und 12 g (0,1 Mol) Acetophenon in 300 ml Toluol wird unter Rühren zu einer Suspension von 2,4 g (0,1 Mol) Natriumhydrid in 100 ml Toluol getropft. Anschließend wird das Gemisch 3 Stunden am Rückfluß gekocht, wobei der entstehende Methylalkohol und etwa die Hälfte des Toluols abdestilliert werden. Der verbleibende Rückstand wird auf eine Mischung von 15 g Eis und 7,2 g Essigsäure gegossen. Man trennt die organische Phase ab, extrahiert die wässrige Phase mit Chloroform (3 mal je 100 ml), vereinigt die organischen Phasen, trocknet sie über Natriumsulfat und dampft im Vakuum ein. Der Rückstand wird über Kieselgel mit Chloroform als Laufmittel fraktioniert, chromatographiert und die entsprechenden Fraktionen aus Legroin umkristallisiert.

Man erhält 17 g (60 % der Theorie) 2-(1,3-Dioxo-3-phenyl-prop-1-yl)-3-methoxycarbonyl-pyridin als weiße Kristalle vom Schmelzpunkt 87 °C.

Beispiel 3

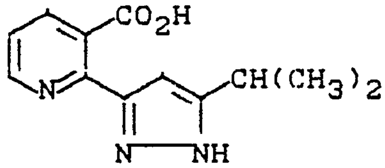

(I-3)

(Verfahren C)

Zu einer Lösung von 10 g (0,04 Mol) 2-(1,3-Dioxo-3-isoprop-1-yl)-3-methoxycarbonyl-pyridin in 150 ml Toluol werden 4 g (0,08 Mol) Hydrazinhydrat getropft. Nach Abklingen der exothermen Reaktion wird 3 Stunden bei 20 °C nachgerührt. Der ausfallende Niederschlag wird abgesaugt und anschliessend mehrfach mit Wasser verrüht. Die wässrigen Extrakte werden im Vakuum eingedampft.

Man erhält 6,6 g (71 % der Theorie) 2-(5-Isopropyl-pyrazol-3-yl)-3-carbonsäure-pyridin als hellgelbe Kristalle vom Schmelzpunkt 203 °C (Zersetzung).

Beispiel 4 und 5:

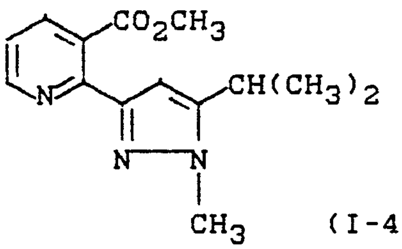

(I-4)

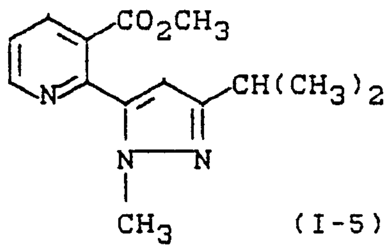

(I-5)

(Verfahren C)

Zu 12,5 g (0,05 Mol) 2-(1,3-Dioxo-3-isoprop-1-yl)-3-methoxycarbonyl-pyridin und 10 g Natriumacetat in 100 ml Eisessig werden 5,3 ml (0,1 Mol) Methylhydrazin zugetropft, wobei das Reaktionsgemisch unter 30 °C gehalten wird. Anschließend wird 1 Stunde unter Rückfluß nachgerührt. Das Reaktionsgemisch wird auf 600 ml Wasser gegossen und mit Essigsäureethylester extrahiert. Das Extrakt wird mit Natriumhydrogencarbonatlösung neutral gewaschen, über Natriumsulfat getrocknet und im Vakuum eingeengt.

Man erhält 12,5 g (96 % der Theorie) eines Öls, das zu 85,5 % aus 2-(5-Isopropyl-1-methyl-pyrazol-3-yl)-3-methoxycarbonyl-pyridin und zu 14,5 % aus 2-(5-Isopropyl-2-methyl-pyrazol-3-yl)-3-methoxycarbonyl-pyridin besteht.

In analoger Weise und entsprechend den erfindungsgemäßen Verfahren werden die nachfolgenden Verbindungen der allgemeinen Formel (I)

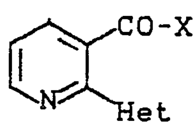

(I)

erhalten:

Tabelle 1

| Bsp. Nr. | X | Het | Physikalische Daten |
|---|---|---|---|
| I-6 | $OCH_3$ | 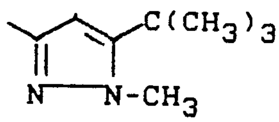 | Öl |
| I-7 | $OCH_3$ | 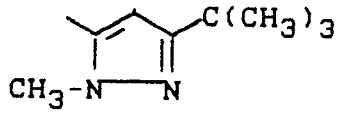 | Öl |
| I-8 | $OCH_3$ | 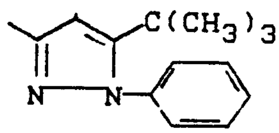 | Öl |
| I-9 | $OCH_3$ | 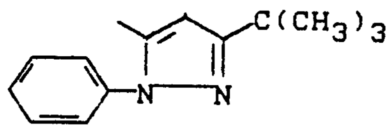 | Öl |
| I-10 | $OCH_3$ | 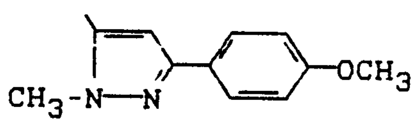 | Fp: 160 °C |
| I-11 | $OCH_3$ | 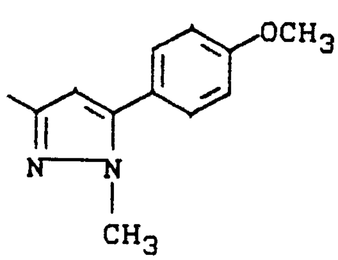 | Fp: 160 °C |
| I-12 | OH | 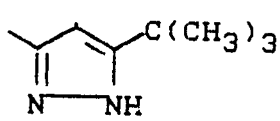 | Fp: 197 °C |

Tabelle 1 (Fortsetzung)

| Bsp. Nr. | X | Het | Physikalische Daten |
|---|---|---|---|
| I-13 | OH | 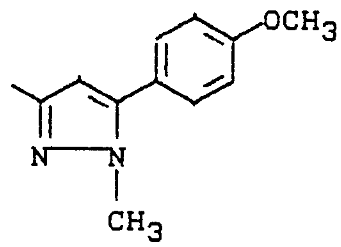 | Fp:194 °C * |
| I-14 | OH | 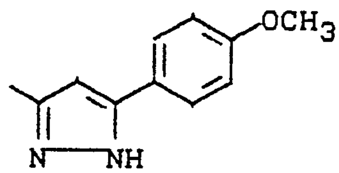 | Fp:148 °C * |
| I-15 | OH | 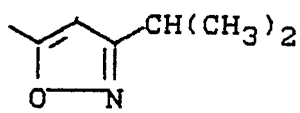 | Fp:123 °C |
| I-16 | OH | 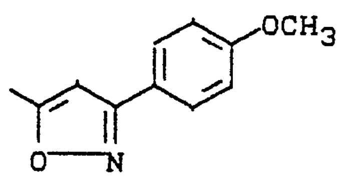 | Fp:203 °C * |
| I-17 | OH | 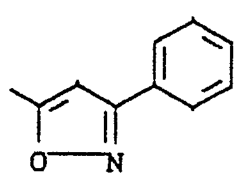 | Fp:211 °C |
| I-18 | OH | 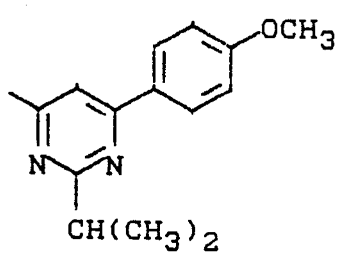 | Fp:155 °C |

* mit Zersetzung

Tabelle 1 (Fortsetzung)

| Bsp. Nr. | X | Het | Physikalische Daten |
|---|---|---|---|
| I-19 | OH | 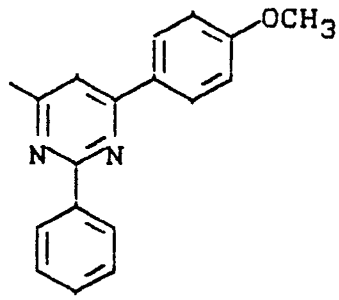 | Fp:200 °C * |
| I-20 | OH | 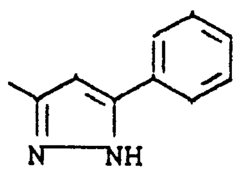 | Fp:205 °C * |
| I-21 | $OCH_3$ | 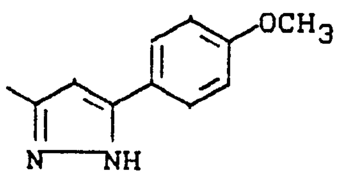 | Fp:110 °C |
| I-22 | $OCH_3$ | 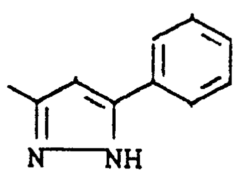 | Fp: 95 °C |
| I-23 | $OCH_3$ | 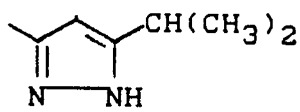 | Öl |

* mit Zersetzung

<u>Tabelle 1</u> (Fortsetzung)

| Bsp. Nr. | X | Het | Physikalische Daten |
|---|---|---|---|
| I-24 | OH | 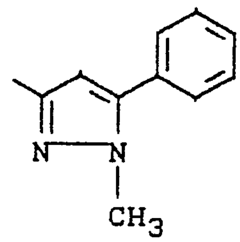 | Fp:140 °C |
| I-25 | $OCH_3$ | 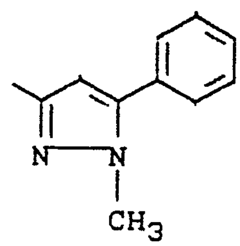 | Öl |
| I-26 | $OCH_3$ | 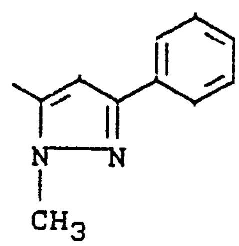 | Öl |
| I-27 | OH | 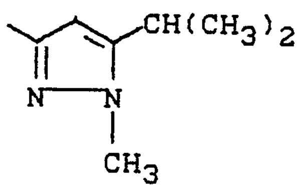 | Fp:147 °C |
| I-28 | OH | 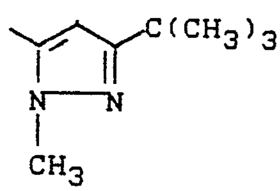 | Fp:203 °C |
| I-29 | OH | 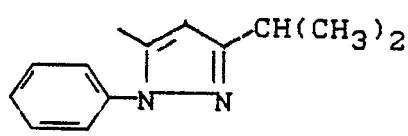 | Fp:168 °C |

Tabelle 1 (Fortsetzung)

| Bsp. Nr. | X | Het | Physikalische Daten |
|---|---|---|---|
| I-30 | $OCH_3$ | 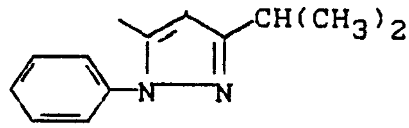 | Öl |
| I-31 | $OCH_3$ | 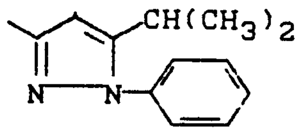 | Öl |
| I-32 | OH | 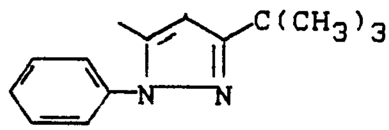 | Fp:115 °C |
| I-33 | $OCH_3$ | 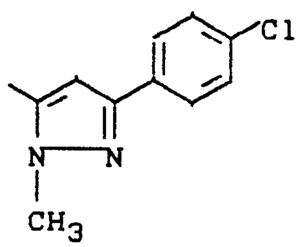 | Fp:145 °C |
| I-34 | $OCH_3$ | 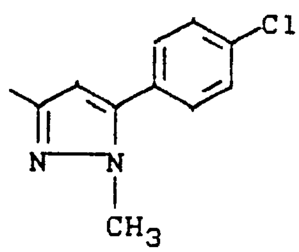 | Fp:145 °C |
| I-35 | $OCH_3$ | 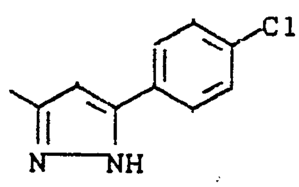 | Fp:167 °C |

* mit Zersetzung

Tabelle 1 (Fortsetzung)

| Bsp. Nr. | X | Het | Physikalische Daten |
|---|---|---|---|
| I-36 | $OCH_3$ | 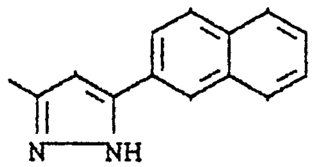 | Fp.: 185° C |
| I-37 | $OCH_3$ | 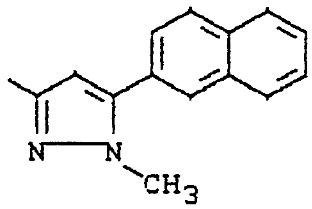 | Fp: 116° C |
| I-38 | OH | 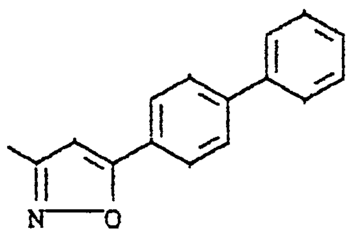 | Fp: 206° C* |
| I-39 | $OCH_3$ | 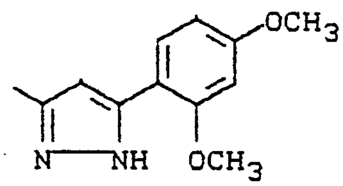 | Fp: 139° C |
| I-40 | $OCH_3$ | 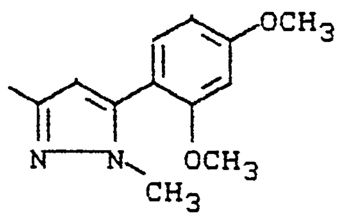 | Öl |
| I-41 | $OCH_3$ | 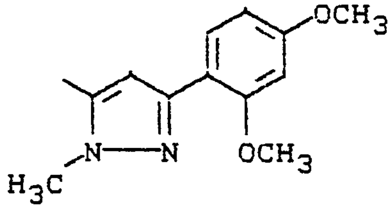 | Öl |

* mit Zersetzung

Tabelle 1 (Fortsetzung)

| Bsp. Nr. | X | Het | Physikalische Daten |
|---|---|---|---|
| I-42 | OH | 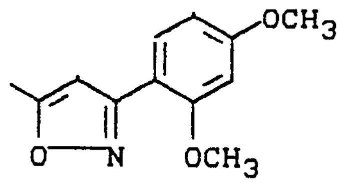 | Fp: 228° C |
| I-43 | OH | 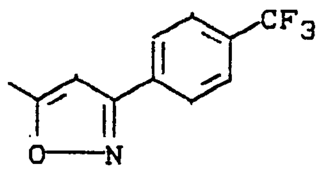 | Fp: 212° C |
| I-44 | OH | 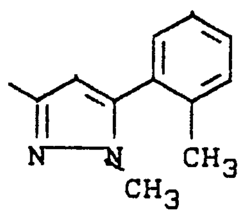 | Fp: 75° C |
| I-45 | OH | 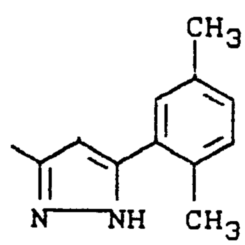 | Fp: 195° C |
| I-46 | OH | 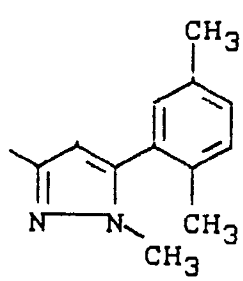 | Fp: 73° C |

<u>Tabelle 1</u> (Fortsetzung)

| Bsp. Nr. | X | Het | Physikalische Daten |
|---|---|---|---|
| I-47 | $OCH_3$ | 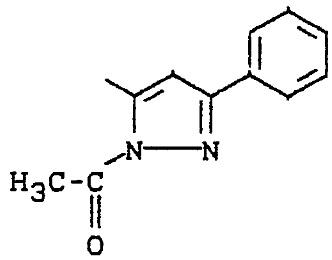 | Fp: 121° C |
| I-48 | $OCH_3$ | 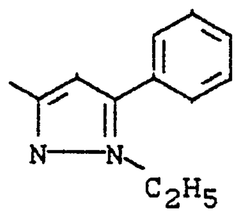 | Fp: 110° C |
| I-49 | OH | 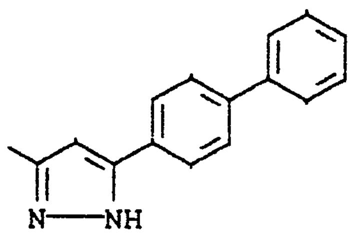 | Fp: 236° C |
| I-50 | OH | 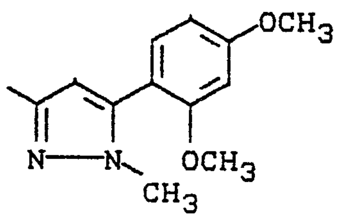 | Fp: 132° C |
| I-51 | $OCH_3$ | 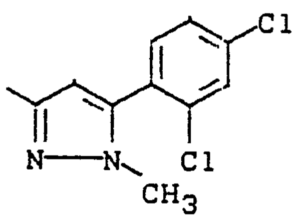 | Fp: 109° C |

Tabelle 1 (Fortsetzung)

| Bsp. Nr. | X | Het | Physikalische Daten |
|---|---|---|---|
| I-52 | OH | 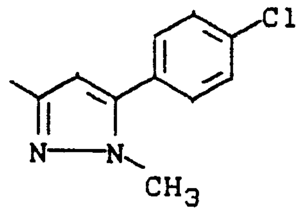 | Fp: 120° C |
| I-53 | $OCH_3$ | 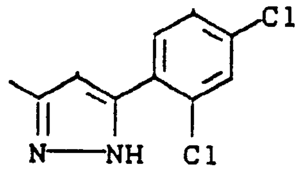 | Fp: 171° C |
| I-54 | $OC_2H_5$ | 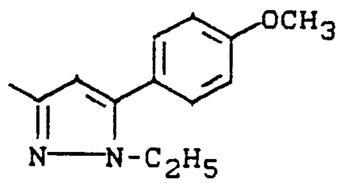 | Fp: 90° C |
| I-55 | $OCH_3$ | 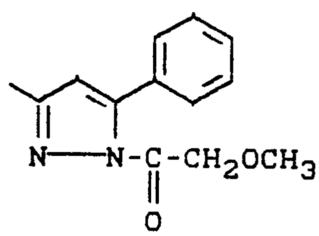 | Öl |
| I-56 | OH | 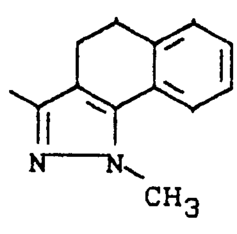 | Fp: 118° C |
| I-57 | OH | 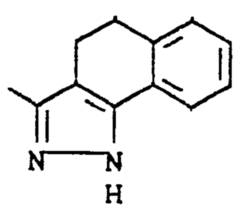 | Fp: 231° C |

Tabelle 1 (Fortsetzung)

| Bsp. Nr. | X | Het | Physikalische Daten |
|---|---|---|---|
| I-58 | $OCH_3$ | 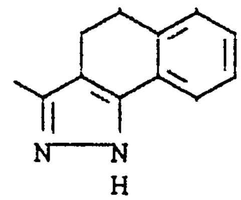 | Fp: 64° C |
| I-59 | $OCH_3$ | 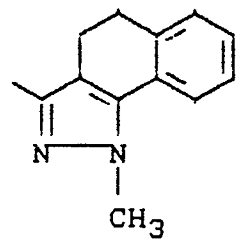 | Fp: 140° C |
| I-60 | $OCH_3$ | 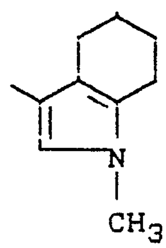 | Fp: 128° C |
| I-61 | $OCH_3$ | 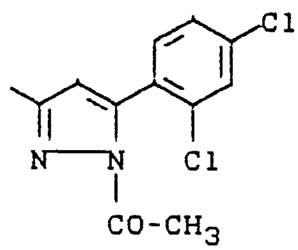 | |
| I-62 | $OCH_3$ | 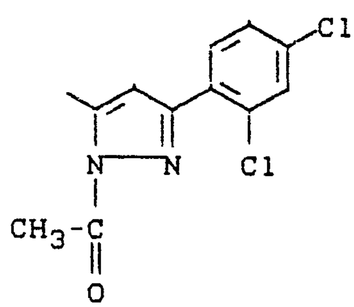 | |

Tabelle 1 (Fortsetzung)

| Bsp. Nr. | X | Het | Physikalische Daten |
|---|---|---|---|
| I-63 | $OCH_3$ | 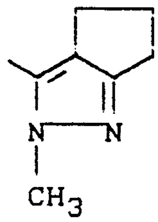 | |
| I-64 | $OCH_3$ | 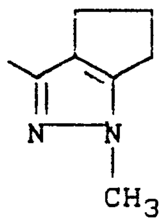 | |
| I-65 | $OCH_3$ | 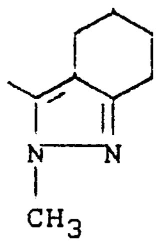 | |

Entsprechend dem Herstellungsbeispiel (II-1) bzw. den vorne angegebenen Verfahrensbedingungen werden die nachfolgenden Ausgangsstoffe der allgemeinen Formel (II)

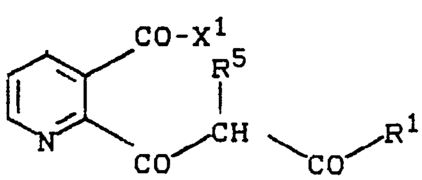

(II)

erhalten:

Tabelle 2

| Bsp. Nr. | $X^1$ | $R^1$ | $R^5$ | Physikalische Daten |
|---|---|---|---|---|
| II-2 | $OCH_3$ | $-CH_2-CO-CH_3$ | H | Öl |
| II-3 | $OCH_3$ | 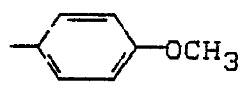 | H | Fp: 89 °C |
| II-4 | $OCH_3$ | $-CH(CH_3)_2$ | H | Öl |
| II-5 | $OCH_3$ | $-C(CH_3)_3$ | H | Öl |
| II-6 | $OCH_3$ | 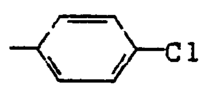 | H | Fp: 99 °C |
| II-7 | $OCH_3$ | 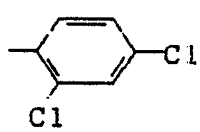 | H | Fp: 85 °C |

Tabelle 2 (Fortsetzung)

| Bsp. Nr. | $X^1$ | $R^1$ | $R^5$ | Physikalische Daten |
|---|---|---|---|---|
| II-8 | $OCH_3$ | 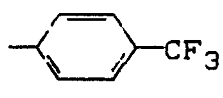 | H | Fp: 198-202° C (Zers.) |
| II-9 | $OCH_3$ | 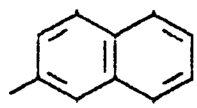 | H | Fp: 106° C |
| II-10 | $OCH_3$ | 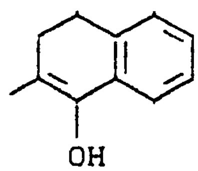 | | Fp: 91° C |
| II-11 | $OCH_3$ | 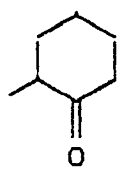 | | Fp: 74° C |
| II-12 | $OCH_3$ | 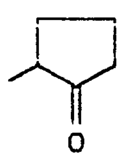 | | Öl |

Beispiel A

Pre-emergence-Test / Gewächshaus

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen boniert in % Schädigung im Vergleich zur Entwicklung der unbehandelten Kontrolle.

In diesem Test zeigt z.B. die Verbindung gemäß Herstellungsbeispiel I-13 eine sehr gute herbizide Wirkung gegen monokotyle und dikotyle Unkräuter.

Beispiel B

Post-emergence-Test/Gewächshaus

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 -15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen boniert in % Schädigung im Vergleich zur Entwicklung der unbehandelten Kontrolle.

In diesem Test zeigt z.B. die Verbindung gemäß Herstellungsbeispiel I-13 eine sehr gute herbizide Wirkung gegen monokotyle und dikotyle Unkräuter.

## Ansprüche

1. Nicotinsäurederivate der allgemeinen Formel (I)

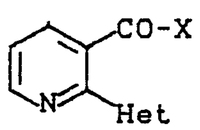

(I)

in welcher
X für Hydroxy, Alkoxy oder den Rest -OM steht, wobei
M für ein Alkali-oder Erdalkaliionenäquivalent oder ein gegebenenfalls substituiertes Ammoniumion steht, und
Het für jeweils substituiertes Isoxazolyl, Pyrimidinyl oder Pyrazolyl steht, wobei jeweils als Substituenten Alkyl, Alkenyl, Alkoxy, Alkylthio, Alkylamino, Hydroxy, Mercapto, Amino, gegebenenfalls durch Halogen oder Alkoxy substituiertes Alkylcarbonyl, Cycloalkyl sowie gegebenenfalls substituiertes Phenyl und Naphthyl in Frage kommen, weiterhin Pyrazolyl einen ankondensierten gegebenenfalls substituierten Cycloalkylrest enhalten kann.

2. Nicotinsäure-Derivate der Formel (I) gemäß Anspruch 1, in welcher
X für Hydroxy, für Alkoxy mit 1 bis 6 Kohlenstoffatomen, oder für den Rest -OM steht, wobei
M für ein Natrium-oder Kaliumion, für ein Magnesium-oder Calciumionenäquivalent oder für ein Ammonium-, Mono-, Di-, Tri-oder Tetraalkylammoniumion mit jeweils 1 bis 4 Kohlenstoffatomen in den einzelnen Alkylresten steht, und
Het für

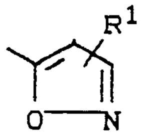 , 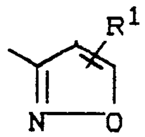 , 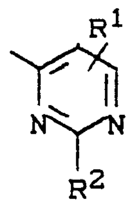 , 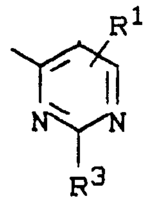 ,

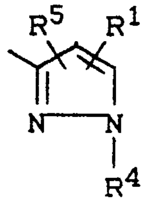 oder 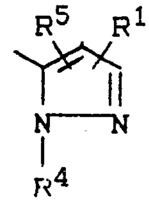

steht, wobei

$R^1$ für geradkettiges oder verzweigtes Alkyl mit 1 bis 6 Kohlenstoffatomen, für Alkenyl mit 2 bis 6 Kohlenstoffatomen, für Cycloalkyl mit 3 bis 6 Kohlenstoffatomen,

für Acetyl sowie für jeweils gegebenenfalls einfach bis dreifach, gleich oder verschieden substituiertes Phenyl oder Naphthyl steht, wobei als Substituenten in Frage kommen:

Halogen, jeweils geradkettiges oder verzweigtes Alkyl, Alkoxy, Alkylthio und Dialkylamino mit 1 bis 4 Kohlenstoffatomen in den einzelnen Alkylteilen, Halogenalkyl mit 1 oder 2 Kohlenstoffatomen und 1 bis 5 gleichen oder verschiedenen Halogenatomen wie Fluor-und Chloratomen, Nitro und Phenyl,

$R^2$ für jeweils geradkettiges oder verzweigtes Alkyl, Alkoxy, Alkylthio oder Alkylamino mit 1 bis 6 Kohlenstoffatomen oder für gegebenenfalls einfach bis dreifahc, gleich oder verschieden substituiertes Phenyl steht, wobei als Substituenten Halogen, geradkettiges oder verzweigtes Alkyl und Alkoxy mit jeweils 1 bis 4 Kohlenstoffatomen und Halogenalkyl mit 1 oder 2 Kohlenstoffatomen und 1 bis 5 gleichen oder verschiedenen Halogenatomen wie Fluor-und Chloratomen in Frage kommen,

$R^3$ für Hydroxy, Mercapto oder für Amino steht,

$R^4$ für Wasserstoff, geradkettiges oder verzweigtes Alkyl mit 1 bis 6 Kohlenstoffatomen, gegebenenfalls durch Fluor, Chlor und/oder Brom oder $C_1$-$C_4$-Alkoxy substituiertes $C_1$-$C_4$-Alkylcarbonyl oder für gegebenenfalls einfach bis dreifach, gleich oder verschieden substituiertes Phenyl steht, wobei als Substituenten Halogen, geradkettiges oder verzweigtes Alkyl und Alkoxy mit jeweils 1 bis 4 Kohlenstoffatomen und Halogenalkyl mit 1 oder 2 Kohlenstoffatomen und 1 bis 5 gleichen oder verschiedenen Halogenatomen wie Fluor-und Chloratomen in Frage kommen,

$R^5$ für Wasserstoff steht oder

$R^1$ und $R^5$ gemeinsam für eine gegebenenfalls benzannellierte Alkylenkette mit 3 bis 5 Kohlenstoffatomen stehen.

3. Nicotinsäure-Derivate der Formel (I) gemäß Anspruch 1, in welcher

X für Hydroxy, Methoxy, Ethoxy, n-oder i-Propoxy oder für den Rest -OM steht, wobei

M für Natrium-, Kalium-, Ammonium-oder Tetraalkylammoniumion mit 1 bis 4 Kohlenstoffatomen in den einzelnen Alkylresten steht, und

Het für

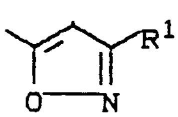 , 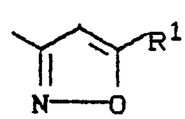 , 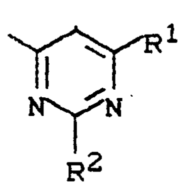 , 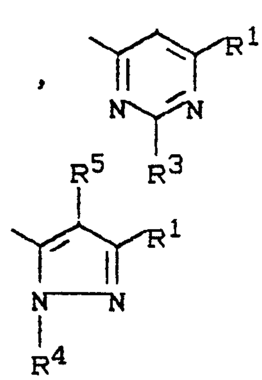 ,

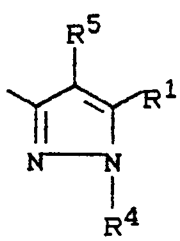 oder

steht, wobei

$R^1$ für Methyl, Ethyl, n-oder i-Propyl, n-, i-, s-oder t-Butyl, für Vinyl oder Isopropyliden, für Cyclopropyl, Cylcopentyl, Cyclohexyl, Acetyl sowie für jeweils gegebenenfalls einfach oder zweifach, gleich oder verschieden substituiertes Phenyl oder Naphthyl steht, wobei als Substituenten in Frage kommen:

Fluor, Chlor, Brom, Iod, Methyl, Ethyl, n-und i-Propyl, Methoxy, Ethoxy, n-und i-Propoxy, Methylthio, Ethylthio, n-und i-Propylthio, Dimethylamino, Diethylamino,Dipropylamino, Trifluormethyl, Nitro und Phenyl,

$R^2$ für Methyl, Ethyl, n-oder i-Propyl, n-, i-, s-oder t-Butyl, Methoxy, Ethoxy, n-oder i-Propoxy, n-, i-, s-oder t-Butoxy, Methylthio, Ethylthio, n-oder i-Propylthio, n-, i-, s-oder t-Butylthio, Methylamino, Ethylamino, n-und i-Propylamino sowie für gegebenenfalls einfach oder zweifach, gleich oder verschieden substituiertes Phenyl steht, wobei als Substituenten in Frage kommen:

Fluor, Chlor, Brom, Methyl, Ethyl n-und i-Propyl, Methoxy, Ethoxy, n-und i-Propoxy und Trifluormethyl,

$R^3$ für Hydroxy, Mercapto oder Amino steht, und

$R^4$ für Wasserstoff, Methyl, Ethyl, n-oder i-Propyl, n-, i-, s-oder t-Butyl, für Acetyl, Chlormethylcarbonyl, Chlorethylcarbonyl, Methoxymethylcarbonyl, Ethoxymethylcarbonyl, Methoxyethylcarbonyl, Ethoxyethylcarbonyl, sowie für gegebenenfalls einfach oder zweifach, gleich oder verschieden substituiertes Phenyl steht, wobei als Substituenten in Frage kommen:

Fluor, Chlor, Brom, Methyl, Ethyl, n-und i-Propyl, Methoxy, Ethoxy, n-und i-Propoxy und Trifluormethyl steht,

$R^5$ für Wasserstoff steht oder

$R^1$ und $R^5$ gemeinsam für eine gegebenenfalls benzannellierte Alkylenkette mit 3 oder 4 Kohlenstoffatomen stehen.

4. Verfahren zur Herstellung von Nicotinsäure-Derivaten der Formel (I),

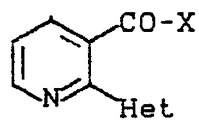

(I)

in welcher

X für Hydroxy, Alkoxy oder den Rest -OM steht, wobei

M für ein Alkali-oder Erdalkaliionenäquivalent oder ein gegebenenfalls substituiertes Ammoniumion steht, und

Het für jeweils substituiertes Isoxazolyl, Pyrimidinyl oder Pyrazolyl steht, wobei jeweils als Substituenten Alkyl, Alkenyl, Alkoxy, Alkylthio, Alkylamino, Hydroxy, Mercapto, Amino, gegebenenfalls durch Halogen oder Alkoxy substituiertes Alkylcarbonyl, Cycloalkyl sowie jeweils gegebenenfalls substituiertes Phenyl oder Naphthyl in Frage kommen, weiterhin pyrazolyl einen ankondensierten gegebenenfalls substituierten Cycloalkylrest enthalten kann,

dadurch gekennzeichnet, daß man

A) Diketone der Formel (IIa),

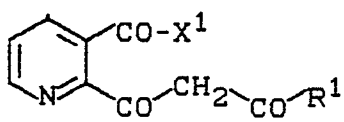

(IIa)

in welcher

$X^1$ für Alkoxy steht und

$R^1$ für Alkyl, Alkenyl, Cycloalkyl, Acetyl sowie für jeweils gegebenenfalls substituiertes Phenyl oder Naphthyl steht,

a) mit Hydroxylamin bzw. dessen Salze zu den Nicotinsäurederivaten der Formel (Ia)

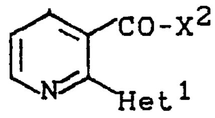

(I a)

in welcher
$X^2$ für Hydroxy oder Alkoxy steht, und
Het' für substituiertes Isoxazolyl steht, wobei als Substituenten Alkyl, Alkenyl, Cycloalkyl, Acetyl sowie jeweils gegebenenfalls substituiertes Phenyl oder Naphthyl in Frage kommen,
oder
b) mit Amidinen der allgemeinen Formel (III), bzw. deren Salze

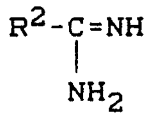

(III)

in welcher
$R^2$ für Alkyl, Alkoxy, Alkylthio, Alkylamino oder gegebenenfalls substituiertes Phenyl steht,
zu den Nicotinsäurederivaten der Formel (Ib),

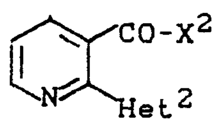

(I b)

in welcher
$X^2$ die oben angegebene Bedeutung hat und
$Het^2$ für substituiertes Pyrimidinyl steht, wobei als Substituenten Alkyl, Alkenyl, Cycloalkyl, Alkoxy, Alkylthio, Alkylamino, Acetyl sowie jeweils gegebenenfalls substituiertes Phenyl oder Naphthyl in Frage kommen,
oder
c) mit Harnstoffderivaten der allgemeinen Formel (IV), bzw. deren Salze
$NH_2$- $\overset{Y}{\overset{\|}{C}}$ -$NH_2$ (IV)
in welcher
Y für Sauerstoff, Schwefel oder den Rest NH steht
zu den Nicotinsäurederivaten der Formel (Ic)

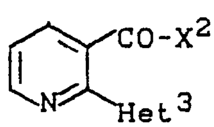

(I c)

in welcher
$X^2$ die oben angegebene Bedeutung hat, und
$Het^3$ für substituiertes Pyrimidinyl steht, wobei als Substituenten Alkyl, Alkenyl, Cycloalkyl, Hydroxy, Mercapto, Amino, Acetyl sowie jeweils gegebenenfalls substituiertes Phenyl oder Naphthyl in Frage kommen,
in Gegenwart eines Verdünnungsmittels umsetzt oder daß man

B) die nach Verfahren A) erhaltenen Nicotinsäurederivate der Formel (Ie)

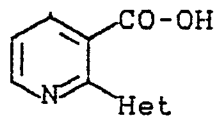

(Ie)

in welcher
Het die oben angegebene Bedeutung hat,
mit einer Base in Gegenwart eines Lösungsmittels zu den Nicotinsäurederivaten der Formel (If)

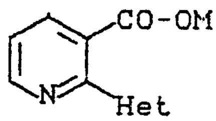

(If)

in welcher
M und Het die oben angegebenen Bedeutungen haben,
umsetzt oder daß man

C) 1,3-Diketone der Formel (II)

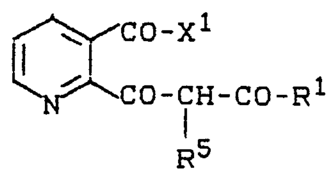

(II)

in welcher
$X^1$ für Alkoxy steht,
$R^1$ für Alkyl, Alkenyl, Cycloalkyl, Acetyl sowie für jeweils gegebenenfalls substituiertes Phenyl oder Naphthyl steht und
$R^5$ für Wasserstoff steht oder
$R^1$ und $R^5$ gemeinsam für eine gegebenenfalls substituierte Alkylenkette stehen,
mit Hydrazinen der allgemeinen Formel (V)
$R^4$-NH-$NH_2$ (V)
in welcher
$R^4$ für Wasserstoff, Alkyl, gegebenenfalls durch Halogen oder Alkoxy substituiertes Alkylcarbonyl oder gegegebenenfalls substituiertes Phenyl steht,
zu den Nicotinsäurederivaten der Formel (Id)

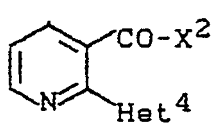

(I d)

in welcher
$X^2$ die oben angegebene Bedeutung hat, und
$Het^4$ für substituiertes Pyrazolyl steht, wobei als Substituenten Alkyl, Alkenyl, Cycloalkyl, gegebenenfalls durch Halogen oder Alkoxy substituiertes Alkylcarbonyl sowie jeweils gegebenenfalls substituiertes Phenyl oder Naphthyl in Frage kommen, weiterhin Pyrazolyl einen unkondensierten gegebenenfalls substituierten Cycloalkylrest enthalten kann,
in Gegenwart eines Verdünnungsmittels umsetzt.

5. Herbizide Mittel, gekennzeichnet durch einen Gehalt an mindestens einem Nicotinsäure-Derivat der Formel (I) gemäß den Ansprüchen 1 bis 4.

6. Verfahren zur Bekämpfung von Unkräutern, dadurch gekennzeichnet, daß man Nicotinsäure-Derivate der Formel (I) gemäß den Ansprüchen 1 bis 4 auf Unkräuter und/oder ihren Lebensraum einwirken läßt.

7. Verwendung von Nicotinsäure-Derivaten der Formel (I) gemäß den Ansprüchen 1 bis 4 zur Bekämpfung von Unkräutern.

8. Verfahren zur Herstellung von herbiziden Mitteln, dadurch gekennzeichnet, daß man Nicotinsäure-Derivate der Formel (I) gemäß den Ansprüchen 1 bis 4 mit Streckmitteln und/oder oberflächenaktiven Substanzen vermischt.

9. Diketone der Formel (II),

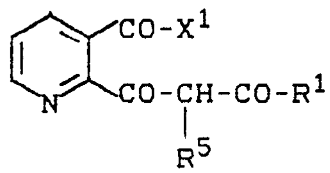

(II)

in welcher
$X^1$ für Alkoxy steht,
$R^1$ für Alkyl, Alkenyl, Cycloalkyl, Acetyl sowie für jeweils gegebenenfalls substituiertes Phenyl oder Naphthyl steht,
$R^5$ für Wasserstoff steht oder
$R^1$ und $R^5$ gemeinsam für eine gegebenenfalls substituierte Alkylenkette stehen.

10. Verfahren zur Herstellung von Diketonen der Formel (II)

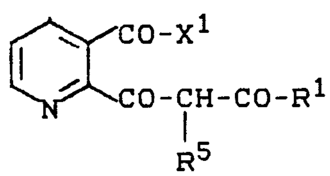

(II)

in welcher
$X^1$ für Alkoxy steht,
$R^1$ für Alkyl, Alkenyl, Cycloalkyl, Acetyl sowie für jeweils gegebenenfalls substituiertes Phenyl oder Naphthyl steht,
$R^5$ für Wasserstoff steht oder
$R^1$ und $R^5$ gemeinsam für eine gegebenenfalls substituierte Alklyenkette stehen.
dadurch gekennzeichnet, daß man Ketone der Formel (VI)
$R^5-CH_2-\overset{O}{\overset{\|}{C}}-R^1$ (VI)
in welcher
$R^1$ und $R^5$ die oben angegebene Bedeutung haben,
mit 2,3-Dialkoxycarbonyl-pyridinen der Formel (VII)

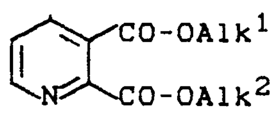

(VII)

in welcher
$Alk^1$ und $Alk^2$ gleich oder verschieden sein können und für Alkyl stehen,
in Gegenwart einer starken Base und in Gegenwart eines Lösungsmittels umsetzt.